# EUROPÄISCHE PATENTSCHRIFT

(11) **EP 2 491 112 B1**
(45) Veröffentlichungstag und Bekanntmachung des Hinweises auf die Patenterteilung: **23.12.2015**
(21) Anmeldenummer: 10765973.2
(22) Anmeldetag: 15.10.2010
(51) Int. Cl.: C12N 1/12, C02F 3/28, C02F 3/32, C12M 1/00, C12P 7/64

(54) **ALGENKULTURVERFAHREN**
PROCESS FOR CULTURING ALGAE
PROCÉDÉ DE CULTURE D'ALGUES

(30) Priorität: 20.10.2009 DE 102009051588
(43) Veröffentlichungstag der Anmeldung: 29.08.2012
(73) Patentinhaber: Fraunhofer-Gesellschaft zur Förderung der angewandten Forschung, 80686 München (DE)
(72) Erfinder: TRÖSCH, Walter, 70329 Stuttgart (DE)
(74) Vertreter: Wohlfahrt, Jan Günther
(86) Internationale Anmeldenummer: PCT/EP2010/006314
(87) Internationale Veröffentlichungsnummer: WO 2011/047809

(56) Entgegenhaltungen:
- EP-A1- 2 105 495
- WO-A1-00/61719
- WO-A1-01/56937
- WO-A1-03/039712
- WO-A1-2009/086307
- WO-A2-02/079484
- US-A- 4 267 038
- US-A- 4 354 936
- US-A1- 2008 050 800
- WILKIE ANN C ET AL: "Recovery of dairy manure nutrients by benthic freshwater algae", BIORESOURCE TECHNOLOGY, Bd. 84, Nr. 1, August 2002 (2002-08), Seiten 81-91, XP002618817, ISSN: 0960-8524
- KEBEDE-WESTHEAD ELIZABETH ET AL: "Production and nutrient removal by periphyton grown under different loading rates of anaerobically digested flushed dairy manure.", JOURNAL OF PHYCOLOGY, Bd. 39, Nr. 6, Dezember 2003 (2003-12), Seiten 1275-1282, XP009143674, ISSN: 0022-3646
- TRAVIESO L ET AL: "Assessment of a microalgae pond for post-treatment of the effluent from an anaerobic fixed bed reactor treating distillery wastewater", ENVIRONMENTAL TECHNOLOGY, Bd. 29, Nr. 9, September 2008 (2008-09), Seiten 985-992, XP009143656, ISSN: 0959-3330
- OSWALD ET AL: "Productivity of algae in sewage disposal", SOLAR ENERGY, PERGAMON PRESS. OXFORD, GB, Bd. 15, Nr. 1, 1. Mai 1973 (1973-05-01), Seiten 107-117, XP023627489, ISSN: 0038-092X, DOI: DOI:10.1016/0038-092X(73)90013-3 [gefunden am 1973-05-01]
- MUNOZ R ET AL: "Algal-bacterial processes for the treatment of hazardous contaminants: A review", WATER RESEARCH, ELSEVIER, AMSTERDAM, NL, Bd. 40, Nr. 15, 1. August 2006 (2006-08-01), Seiten 2799-2815, XP025039987, ISSN: 0043-1354, DOI: DOI:10.1016/J.WATRES.2006.06.011 [gefunden am 2006-08-01]
- WANG L ET AL: "Anaerobic digested dairy manure as a nutrient supplement for cultivation of oil-rich green microalgae Chlorella sp", BIORESOURCE TECHNOLOGY, ELSEVIER BV, GB, Bd. 101, Nr. 8, 1. April 2010 (2010-04-01) , Seiten 2623-2628, XP026833834, ISSN: 0960-8524 [gefunden am 2009-11-24]

## Beschreibung

Die vorliegende Erfindung betrifft die weitere Aufreinigung wässriger Phasen von anaerob biologisch gereinigten organischen Suspensionen. Die vorliegende Erfindung betrifft auch die Kultivierung von Algen, insbesondere Mikroalgen.

Die vorliegende Erfindung betrifft bioprozesstechnische Verfahren, bei denen wässrige Phasen anaerob biologisch gereinigter organischer Suspensionen als Medienbestandteile in Algenkulturen zugeführt werden. Die vorliegende Erfindung betrifft auch die erfindungsgemäßen Verfahren in der Ausgestaltung als Herstellverfahren. Die vorliegende Erfindung betrifft auch die Verwendung von wässrigen Phasen von anaerob biologisch gereinigten organischen Suspensionen als Medienbestandteile von Algenkulturen. Die vorliegende Erfindung betrifft auch die Verwendung von wässrigen Phasen von anaerob biologisch gereinigten organischen Suspensionen zur Verbesserung der Wachstumsbedingungen von Algen in Photobioreaktoren. Die vorliegende Erfindung betrifft auch die Verwendung von Algen zur Reinigung von wässrigen Phasen von anaerob biologisch gereinigten organischen Suspensionen, insbesondere von anaerob biologisch gereinigtem Abwasserfiltrat. Die vorliegende Erfindung betrifft auch bioprozesstechnische Vorrichtungen, enthaltend einen Bioreaktor, insbesondere einen Faulturm, und einen Photobioreaktor, sowie die Verwendung dieser Vorrichtungen zu den erfindungsgemäßen Verfahren.

Vorrichtungen und Verfahren zur Reinigung von Abwasser, vor allem von kommunalen Abwässern, sind bekannt. Beispiele dafür sind die sogenannten Kläranlagen beziehungsweise Abwasserreinigungsanlagen. Diese dienen primär der Reinigung kommunaler Abwässer, die von der öffentlichen Kanalisation gesammelt und zur Kläranlage transportiert werden. Das Ziel der Abwasserreinigung ist die Entfernung unerwünschter Bestandteile aus dem Abwasser, sodass ein gereinigtes Abwasser erhalten wird, das beispielsweise in Flüsse und Gewässer (so genannte Vorfluter) freigesetzt werden kann, ohne dass Schäden für die Umwelt und die Bevölkerung durch chemische oder mikrobiologische Belastung auftreten.

Zur Entfernung der unerwünschten Bestandteile werden, meist in Kombination, mechanische (physikalische), biologische und chemische Verfahren eingesetzt. Moderne Kläranlagen sind dementsprechend mindestens dreistufig, wobei mindestens eine physikalische Reinigungsstufe, mindestens eine biologische Reinigungsstufe und mindestens eine chemische Reinigungsstufe hintereinander geschaltet sind.

Biologische Verfahren dienen vor allem dem Abbau von organischen Verbindungen in organisch hoch belasteten Abwässern. Dies erfolgt häufig durch aeroben Abbau der organischen Verbindungen zu anorganischen Endprodukten wie Kohlenstoffdioxid, Nitrat, Sulfat, Phosphat, etc. Dazu wird Biomasse in Form von Belebtschlämmen in sogenannten Belebungsbecken beziehungsweise als Bakterienrasen zum Beispiel in Form sogenannter Tropfkörper eingesetzt. Die zu entfernenden organischen Verbindungen werden dabei nicht nur durch katabolische Vorgänge energetisch verwertet und "veratmet", d.h. durch Umsetzung mit Luftsauerstoff abgebaut, sondern dienen in anabolischen Vorgängen dem Wachstum der Biomasse. Die sich anreichernde Biomasse muss regelmäßig aus der biologischen Reinigungsstufe entfernt und in einem Faulturm einer Nachbehandlung, zum Beispiel einer Vergärung unterzogen werden, wobei Biogas freigesetzt und trotzdem noch erhebliche Mengen Schlammabfall überbleiben. Dieser Schlammabfall kann teilweise als Dünger verwendet werden. Zum Großteil wird das Schlammaufkommen jedoch kostenträchtig in Verbrennungsanlagen beseitigt.

Bekannt ist auch eine anaerobe biologische Abwasserreinigung. Diese dient ebenfalls der Entfernung schädlicher oder störender organischer Kohlenstoffverbindungen im Abwasser durch mikrobiologische Abbauprozesse, die jedoch in Abwesenheit von Sauerstoff ablaufen. Dabei gewinnen die anaeroben Mikroorganismen die für ihren Stoffwechsel erforderliche Energie aus der Umsetzung der organischen Kohlenstoffverbindungen und setzen diese zu organischen Säuren, anderen Kohlenwasserstoffen und zu Kohlendioxid und Methan um. Die Abwasserreinigung findet dabei in luftdicht verschlossenen Reaktoren statt.

Allgemein unterteilt man Anaerobverfahren in Durchflusssysteme ohne Immobilisierung der Biomasse, worin die Biomasse im Wesentlichen in Suspension vorliegt, und in Durchflusssysteme mit Immobilisierung, worin die Biomasse im Wesentlichen an innere Strukturen des Reaktors immobilisiert vorliegt und von dem zu reinigenden Abwasser umströmt wird.

Bekannt ist beispielsweise der sogenannte "Emscherbrunnen", ein von Immhoff 1906 patentierter zweistufiger Klärturm der im unten liegenden Faulraum eine anaerobe Faulung ermöglicht.

Neben Faultürmen werden zur bekannten anaeroben Abwasserreinigung auch Fließbettreaktoren, anaerobe Belebungsbecken, UASB-Reaktoren oder Festbettreaktoren eingesetzt.

Es werden aber nicht nur kommunale Abwässer und Klärschlämme sondern auch andere Abwässer anaerob behandelt, beispielsweise Abwässer aus der Industrie, insbesondere Lebensmittelindustrie, oder Abwässer aus der Vergärung nachwachsender Rohstoffe. Beispielsweise entsteht bei der Verarbeitung von Zuckerrüben in Zuckerfabriken hochbelastetes Abwasser, welches durch bekannte anaerobe Abwasserreinigungsanlagen gereinigt wird. Dabei werden die Abwässer zweistufig abgebaut: In einer ersten Stufe, der sogenannten Versäuerung, werden die im Abwasser enthaltenen hochmolekularen Zuckerstoffe und andere organische Verbindungen durch Mikroorganismen zu organischen Säuren umgebaut. Diese Säuren sind das abbaubare Substrat für die Bakterien in der zweiten Stufe, der sogenannten methanogenen Phase. Die methanogene Phase ist dabei die mikrobielle Phase, die das Abwasser reinigt, indem CSB (gelöste oxidierbare organische Substanz) zu gasförmigen Produkten CH₄ und CO₂ umgewandelt werden, die als solche das Wasser kostenlos und freiwillig verlassen.

Die bei bekannten anaeroben Reinigungsverfahren erreichten Restkonzentrationen organischer Verunreinigungen erlauben keine direkte Einleitung in den Vorfluter. Es sind daher normalerweise weitere aerobe biologische, physikalische und/oder chemische Reinigungsstufen erforderlich.

Verfahren und Vorrichtungen, die der anaeroben biologischen Reinigung von Abwasser dienen, wobei gleichzeitig Biogas gewonnen werden kann, sind beispielsweise aus der DE 10 2005 063 228 A1 bekannt.

Das anaerob gereinigte Abwasser enthält auch Biomasse, insbesondere Bakterien, in einer Konzentration, die keine direkte Einleitung in den Vorfluter erlauben. Daher wird diese Biomasse oft mittels physikalischer Verfahren, insbesondere mittels Filtration, aus dem Abwasser entfernt. Bei dieser Filtration werden insbesondere mit Hilfe von Membranen durchgeführte Trennprozesse angewendet. Trennschritte in der Membrantrenntechnik lassen sich nach den Trenngrenzen in die Klassen Mikro-, Ultra- und Nanofiltration sowie Umkehrosmose einteilen. Beim Trennvorgang wird die Biomasse als Feststoff zumindest direkt an der Membran aufkonzentriert, während die filtrierte Flüssigkeit die Membran passiert.

Es sind verschiedene Filtersysteme zur Nachbehandlung von anaerob gereinigten Abwässern bekannt. Die DE 100 04 096 A1 und die DE 10154549 A1 beschreiben zum Beispiel Filtersysteme, die sich zur Mikrofiltration oder Ultrafiltration von anaerob behandelten Abwässern eignen. Häufig wird eine dynamische Filtration angewendet, bei der in einem Eindickungsprozess ein Konzentrat (Retenat) und ein Filtrat (Permeat) erhalten werden. Das Konzentrat enthält dabei die suspendierten, dispergierten oder emulgierten Teilchen sowie der gelösten Substanzen entsprechend dem Trennvermögen des ausgewählten Filtersystems, beispielsweise der ausgewählten Membran. Das Konzentrat wird häufig in den anaeroben Reinigungsschritt zurückgeleitet. Das Filtrat ist die aufgearbeitete Flüssigkeit, frei von den Partikeln, deren Größen oberhalb der Trenngrenze des Filtersystems liegen.

Obwohl die Reinigungseffektivität beim Abbau organischer Verunreinigungen mit solchen anaeroben biologischen Reinigungen von Abwässern, insbesondere in Kombination mit einer anschließenden Filtrierung hoch ist, enthalten die so gereinigten Abwässer noch hohe Mengen an Phosphat und Stickstoff. Daher ist eine umweltschonende Entsorgung dieser Abwässer notwendig, um Vorfluter nicht mit großen Mengen an Phosphat und Stickstoff zu belasten.

Die anaerobe Reinigung von Abwasser führt zur Produktion von Biogas. Die anaerobe Reinigung von Abwasser führt zur Produktion von großen Mengen CO₂. Auch werden große Mengen Methan erhalten, das unter Bildung von weiterem CO₂ zur Energiegewinnung verbrannt werden kann. Um die Freisetzung von CO₂ in umweltschädlichen Mengen zu reduzieren, wenn nicht gar zu verhindern, muss also entweder die Produktion von CO₂ verringert werden oder das gebildete CO₂ wird umweltverträglich gebunden oder umgewandelt. Dabei ist die Photosynthese eine der wenigen CO₂-verbrauchenden Prozesse in der Natur. Aus CO₂, Wasser und Licht als Energiequelle wird CO₂ in Pflanzen und Algen fixiert, dabei entsteht Wasser und Sauerstoff. Jährlich werden über die Photosynthese global in etwa 360 Gt CO₂ fixiert. In den Weltmeeren fixieren Mikroalgen dabei etwa 175 Gt CO₂. Diese Mikroalgen bilden in den Weltmeeren die Basis der Nahrungskette.

Die photosynthetische Biomasseproduktion kann signifikant zur Reduzierung des CO₂-Gehalts der Atmosphäre beitragen, wenn sie sowohl als Kohlenstoffspeicher als auch Substitut für fossile Brennstoffe oder fossile Rohstoffe dient. Da bei einer energetischen Nutzung von photosynthetisch hergestellter Biomasse nur soviel CO₂ freigesetzt wird, wie während des Wachstums photosynthetisch gebunden wurde, wird unter der Voraussetzung eines nachhaltigen Prozesses CO₂ eingespart, wenn ein mit fossilen Rohstoffen hergestelltes Produkt ersetzt wird.

Algen, insbesondere Mikroalgen, zeichnen sich aufgrund ihrer geringen Größe bzw. Feinverteilung durch eine höhere Photosyntheseeffizienz im Vergleich zu höheren Pflanzen aus. Diese bessere Ausnützung des vorhandenen Sonnenlichts führt zu höheren Biomasseerträgen als bei Landpflanzen. Mikroalgen in Photobioreaktoren haben eine bis zu zehnfach höhere Biomasseproduktivität, die mit einem erhöhten CO₂-Verbrauch einhergeht, als Landpflanzen. Darüber hinaus können Mikroalgen eine Vielzahl hochwertiger lipophiler oder hydrophiler Substanzen produzieren, wie Vitamine, Farbpigmente, Fettsäuren, Aminosäuren und pharmazeutisch-wirksame Stoffe, beispielsweise Antibiotika. Wichtige Stoffklassen sind hierbei zum Beispiel essentielle Fettsäuren, Lipide, Sterole und Karotinoide, Polysaccharide, Proteine oder Aminosäuren und Phycobiliproteine (Pigmente) sowie die Gesamtbiomasse als Protein-reicher, Nucleinsäure-armer Rohstoff. Diese Produkte können als hochwertige Nahrungsmittel, Lebensmittelergänzungsstoffe, Tiernahrung, Pharmazeutika oder als Ersatzstoffe für synthetische Stoffe in Kosmetik- und Chemieindustrie eingesetzt werden.

Zu den Mikroalgen zählen einerseits die prokaryotischen Cyanobakterien als auch eukaryotische mikroskopische Algenklassen.

Kennzeichnend für Mikroalgen-Biomasse ist im Vergleich zu höheren Landpflanzen das Fehlen von Lignin, ein geringer Cellulose- und Nucleinsäuregehalt und ein hoher Kohlenhydrat- und Proteinanteil von bis zu 60 % der Trockensubstanz.

Die Nutzung von CO₂ als Abfallprodukt ist eine Grundvoraussetzung, um Mikroalgen mit Sonnenlicht unter Nettoenergie-Gewinnungsbedingungen herstellen zu können, so dass eine stofflichenergetische bzw. sogar eine rein energetische Verwertung von Algenbiomasse nachhaltig wird. Damit kann ein Beitrag zur Reduktion des atmosphärischen CO₂ aus der Verbrennung fossiler Energieträger geleistet werden. Dies wird schon in einigen Pilotanlagen in Verbindung mit der Verbrennung unterschiedlicher fossiler Energieträger versucht.

Mikroalgen benötigen für ihr Wachstum neben solarer Energie und CO₂ wie jede andere Pflanze auch mineralische Wertstoffe wie Stickstoff und Phosphor sowie andere Elemente, beispielsweise Spurenelemente. Diese werden normalerweise in Form von synthetischen Kulturmedien vorgelegt. Zum Teil wird dabei auf Flüssigdünger für landwirtschaftliche Produkte zurückgegriffen, die auf Blumenbeziehungsweise Kulturpflanzenbedarf zugeschnitten sind. Die mineralischen Bestandteile sind ein wesentlicher Faktor der Produktionsbetriebskosten bei der Kultivierung von Mikroalgen, da Sonnenlicht und CO₂ kostenlos zur Verfügung stehen.

Die Wirtschaftlichkeit der durch Mikroalgen produzierten Biomasse im Allgemeinen und der spezifischen Stoffe im speziellen werden zunächst durch die Produktivität der ausgesuchten Algenspezies bestimmt. Jedoch nur, wenn gleichzeitig ein hoher Umwandlungswirkungsgrad von solarer Strahlungsenergie in die gewünschte Biomasseform erreicht wird und der energetische Aufwand und die Kosten für Herstellung, Installation und Betrieb der Anlage äußerst gering gehalten werden. Hohe Biomasseproduktivität ist ein Problem der optimalen Lichtverteilung pro Volumen. Die Absorption von Licht durch Algen führt zu starker Lichtabnahme mit zunehmender Schichtdicke, gleichzeitig findet eine gegenseitige Selbstbeschattung statt. Genügend hohe Umwandlungswirkungsgrade der solaren Strahlungsenergie können mit Photobioreaktoren erreicht werden. Photobioreaktoren sind Fermenter, in denen phototrophe Mikroorganismen, insbesondere Algen, Cyanobakterien und Purpurbakterien kultiviert werden, in denen also entweder das Wachstum und die Vermehrung dieser Zellen ermöglicht wird oder die Produktion unterschiedlicher Substanzen mittels phototropher Zellen gefördert wird. Ein für die Kultivierung von Mikroalgen besonders geeigneter Photobioreaktor ist aus der DE 199 16 597 A1 bekannt.

Die EP 2 105 495 A1 beschreibt Verfahren und Vorrichtungen für die Umwandlung von Biomasse, insbesondere Jauche, wobei die Biomasse einem anaeroben Abbau unterzogen und die so erhaltene Flüssigkeit einer Filtration, zum Beispiel einer Mikro- oder Ultrafiltration, unterzogen wird, um Mikroorganismen abzutrennen. Die erhaltene Suspension wird einer Algenkultur zugefüttert.

Ein der vorliegenden Erfindung zugrundeliegendes technische Problem ist die Bereitstellung von Verfahren, insbesondere kostengünstigen Verfahren, zur Entfernung hoher Mengen an Stickstoff und Phosphor aus anaerob biologisch gereinigten Abwässern.

Der Erfindung lag auch das technische Problem zugrunde kostengünstige und einfache Kulturmedien für Algen und Mikroalgen, insbesondere bei der Kultivierung in Photobioreaktoren zur Verfügung zu stellen.

Der Erfindung lag auch das technische Problem zugrunde, Verfahren und Vorrichtungen zur Verfügung zu stellen, die es ermöglichen, die durch anaerobe Reinigung von Abwässern und Biomüll erhaltenen Abbauprodukte zu verwerten, insbesondere kostengünstig und energetisch günstig zu verwerten.

Der vorliegenden Erfindung lag auch das technische Problem zugrunde, Verfahren und Vorrichtungen bereitzustellen, die einen energetisch günstigen Kreislauf von anaerobem Abbau von organischen Verbindungen und photosynthetischem Aufbau von organischen Verbindungen erlauben.

Der vorliegenden Erfindung lag auch das technische Problem zugrunde, bei der Reinigung von Abwässern entstehende Abfallprodukte, insbesondere die wässrigen Phasen von anaerob biologisch gereinigten organischen Suspensionen ökologisch und ökonomisch sinnvoll zu verwerten.

Die vorliegende Erfindung löst das ihr zugrunde liegende technische Problem insbesondere auch durch die Bereitstellung eines Verfahrens, insbesondere bioprozesstechnischen Verfahrens, enthaltend den Schritt: Zuführen mindestens einer wässrigen Phase mindestens einer anaerob biologisch gereinigten organischen Suspension als Medienbestandteil in eine Algenkultur.

Die vorliegende Erfindung löst das ihr zugrunde liegende technische Problem also insbesondere auch durch die Bereitstellung eines bioprozesstechnischen Verfahrensgemäß Anspruch 1. In einem Hauptverfahrensschritt wird mindestens eine wässrige Phase mindestens einer anaerob biologisch gereinigten organischen Suspension als Medienbestandteil einer Algenkultur, insbesondere Mikrolagenkultur, zugeführt.

Unter "Hauptverfahrensschritt" wird der für die vorliegende Erfindung wichtige Verfahrensschritt des Zuführens der mindestens einen wässrigen Phase von mindestens einer anaerob biologisch gereinigten organischen Suspension als Medienbestandteil in eine Algenkultur verstanden.

Neben diesem Hauptverfahrensschritt können in dem erfindungsgemäßen Verfahren auch weitere Verfahrensschritte vorgesehen sein. Diese zusätzlichen Verfahrensschritte können vor oder nach dem Hauptverfahrensschritt erfolgen, sie können also als Verfahrensschritte dem Hauptverfahrensschritt vorgeordnet oder nachgeordnet sein.

Erfindungsgemäß bevorzugt wird in dem Hauptverfahrensschritt die wässrige Phase der anaerob biologisch gereinigten organischen Suspension als Medienbestandteil einer Algenkultur zugeführt.

Im Zusammenhang mit der vorliegenden Erfindung wird unter einem bioprozesstechnischen Verfahren jedes Verfahren verstanden, bei dem biologische und/oder biochemische Verfahrensschritte zur Umsetzung und/oder Produktion von Stoffen angewandt werden.

Im Zusammenhang mit der vorliegenden Erfindung wird unter einer anaerob biologisch gereinigten organischen Suspension eine anaerob biologisch gereinigte Suspension verstanden, die organische Anteile enthält.

Im Zusammenhang mit der vorliegenden Erfindung wird unter einer anaerob biologisch gereinigten organischen Suspension ein heterogenes Stoffgemisch aus einer Flüssigkeit, beispielsweise Wasser, und darin fein verteilten, insbesondere organischen Feststoffen verstanden, wobei die Suspension oder zumindest die festen, insbesondere organischen Bestandteile der Suspension einer anaeroben biologischen Reinigung unterzogen wurden. Vor, während oder nach dieser Reinigung kann dann Flüssigkeit, insbesondere Wasser, zugesetzt werden, so dass eine Suspension erhalten wird.

Im Zusammenhang mit der vorliegenden Erfindung wird unter einer wässrigen Phase der anaerob biologisch gereinigten organischen Suspensionen ein flüssiger Anteil dieser Suspensionen verstanden, der hauptsächlich Wasser und darin gelöste Stoffe enthält, aber auch weitere Flüssigkeiten enthalten kann. Die wässrige Phase kann durch Abtrennen von der festen Phase oder von einem Gemisch aus Flüssigkeit und Feststoffen (Konzentrat), beispielsweise durch Filtration gewonnen werden. Die wässrige Phase kann somit einen Teil der Flüssigkeit oder die gesamte Flüssigkeit der anaerob biologisch gereinigten organischen Suspensionen enthalten.

In einer bevorzugten Ausführungsform wird das von der wässrigen Phase abgetrennte Konzentrat nicht als Medienbestandteil der Algenkultur verwendet. Die Konzentrate werden also bevorzugt nicht der Algenkultur zugeführt. In einer bevorzugten Ausführungsform wird die wässrige Phase nicht weiter durch einen Aerob-Tricklingfilter aufgereinigt.

In einer bevorzugten Ausführungsform enthält das Filtrat weniger als 1 Gew.-% an Feststoffen. In einer bevorzugten Ausführungsform enthält das Filtrat nur Feststoffe in gelöster Form. In einer bevorzugten Ausführungsform enthält das Filtrat weniger als 1 Gew.-% an Feststoffen, wobei die Feststoffe ausschließlich in gelöster Form im Filtrat vorliegen.

Die wässrige Phase wird in einem in einem zusätzlichen Schritt vor dem Zuführen in eine Algenkultur aus der anaerob biologisch gereinigten organischen Suspension abgetrennt. Es wird also dem Hauptschritt ein Abtrennschritt vorangestellt, in dem die wässrige Phase von den übrigen Bestandteilen der anaerob biologisch gereinigten organischen Suspension abgetrennt wird. Erfindungsgemäß handelt es sich um eine physikalische Abtrennung.

Erfindungsgemäß bevorzugt ist die anaerobe biologische Reinigung eine anaerobe Vergärung.

In einer bevorzugten Ausführungsform stammt die wässrige Phase aus einem Methangärungsprozess.

In einer Ausführungsform ist die anaerobe biologische Reinigung vor der Durchführung des erfindungsgemäßen Verfahrens bereits erfolgt. Dann ist die anaerobe biologische Reinigung kein Verfahrensschritt des erfindungsgemäßen Verfahrens. In einer Ausführungsform ist die anaerobe biologische Reinigung vor der Durchführung des erfindungsgemäßen Verfahrens bereits erfolgt. Dann ist die anaerobe biologische Reinigung kein Verfahrensschritt des erfindungsgemäßen Verfahrens. In einer alternativen Ausführungsform ist die anaerobe biologische Reinigung ein Verfahrensschritt des erfindungsgemäßen Verfahrens, der dem Hauptverfahrensschritt vorgeordnet ist.

In einer Ausführungsform wird die anaerobe biologische Reinigung in einem zusätzlichen Schritt vor dem Zuführen der wässrigen Phase zu der Algenkultur als einstufige oder zweistufige Methanisierung durchgeführt. In einer Ausführungsform wird die anaerobe biologische Reinigung in einem zusätzlichen Schritt vor dem Zuführen der wässrigen Phase zu der Algenkultur als einstufige Methanisierung durchgeführt. In einer Ausführungsform wird die anaerobe biologische Reinigung in einem zusätzlichen Schritt vor dem Zuführen der wässrigen Phase zu der Algenkultur als zweistufige Methanisierung durchgeführt. In einer Ausführungsform wird die anaerobe biologische Reinigung in einem zusätzlichen Schritt vor dem Zuführen der wässrigen Phase zu der Algenkultur als mehrstufige Methanisierung durchgeführt.

In einer Ausführungsform wird die anaerobe biologische Reinigung in einem zusätzlichen Schritt vor dem Zuführen der wässrigen Phase zu der Algenkultur als Methanisierung in Kaskaden durchgeführt. Die Methanisierung wird also bevorzugt in in Reihe geschalteten Bioreaktoren, beispielsweise Faultürmen, durchgeführt.

Erfindungsgemäß bevorzugt handelt es sich bei den ursprünglichen, insbesondere ungereinigten organischen Suspensionen um Klärschlamm, Biomüll, Abwässer aus der Lebensmittelindustrie, Abwässer aus der kommunalen Abwasserentsorgung und/oder Abwässer aus der Vergärung nachwachsender Rohstoffe. Erfindungsgemäß bevorzugt handelt es sich bei den ursprünglichen, insbesondere ungereinigten organischen Suspensionen um Klärschlamm, Biomüll und/oder Abwässer. Erfindungsgemäß bevorzugt handelt es sich bei den ursprünglichen, insbesondere ungereinigten organischen Suspensionen um Klärschlamm und/oder Abwässer.

Erfindungsgemäß ist die wässrige Phase ein Filtrat, also eine filtrierte, klare Flüssigkeit. Erfindungsgemäß bevorzugt ist die wässrige Phase ein Filtrat der anaerob biologisch gereinigten organischen Suspension. Erfindungsgemäß ist die wässrige Phase ein Sterilfiltrat. Erfindungsgemäß wird die anaerob biologisch gereinigte organische Suspension und/oder die wässrige Phase in einem zusätzlichen Schritt vor dem Zuführen zu der Algenkultur mit einem Filter mindestens einmal gefiltert.

Es wird also in einem Schritt a) die organische Suspension anaerob biologisch gereinigt, in einem Schritt b) die anaerob biologisch gereinigte organische Suspension gefiltert und in einem Schritt c) die durch das Filtern gewonnene wässrige Phase der anaerob biologisch gereinigten organischen Suspension als Medienbestandteil einer Algenkultur zugeführt.

Erfindungsgemäß werden die anaerob biologisch gereinigte organische Suspension und/oder die wässrige Phase in einem zusätzlichen Schritt vor dem Zuführen in die Algenkultur mit mindestens ein Filtrationsvorrichtung, insbesondere Mikrofiltrationsvorrichtung, ausgewählt aus einem Rotationsscheibenfilter, einem Polymerfilter und einem keramischen Tiefenfilter mindestens einmal gefiltert.

In einer erfindungsgemäßen Ausführungsform werden die anaerob biologisch gereinigte organische Suspension und/oder die wässrige Phase in einem zusätzlichen Schritt vor dem Zuführen in die Algenkultur mit mindestens ein Filtrationsvorrichtung, insbesondere Mikrofiltrationsvorrichtung, ausgewählt aus einem Rotationsscheibenfilter, einem Polymerfilter und einem keramischen Tiefenfilter mindestens einmal dynamisch gefiltert.

In einer erfindungsgemäßen Ausführungsform werden die anaerob biologisch gereinigte organische Suspension und/oder die wässrige Phase in einem zusätzlichen Schritt vor dem Zuführen in die Algenkultur mit einem Rotationsscheibenfilter, einem Polymerfilter oder einem keramischen Tiefenfilter mindestens einmal gefiltert. In einer erfindungemäßen Ausführungsform wird die anaerob biologisch gereinigte organische Suspension und/oder die wässrige Phase in einem zusätzlichen Schritt vor dem Zuführen in die Algenkultur mit einem Rotationsscheibenfilter mindestens einmal gefiltert. Erfindungsgemäß bevorzugt wird zum Filtern ein Rotationsscheibenfilter, ein Polymerfilter oder ein keramischer Tiefenfilter verwendet. In einer erfindungsgemäßen Ausführungsform wird zum Filtern ein Rotationsscheibenfilter verwendet. In einer erfindungsgemäßen Ausführungsform wird zum Filtern ein Polymerfilter verwendet. In einer erfindungsgemäßen Ausführungsform wird zum Filtern ein keramischer Tiefenfilter verwendet. In einer erfindungsgemäßen Ausführungsform wird zum Filtern ein dynamischer Rotationsscheibenfilter verwendet. In einer erfindungsgemäßen Ausführungsform wird zum Filtern ein dynamischer Polymerfilter verwendet. In einer erfindungsgemäßen Ausführungsform wird zum Filtern ein dynamischer keramischer Tiefenfilter verwendet.

In einer erfindungsgemäßen Ausführungsform wird ein dynamisches Filtrierungsverfahren verwendet.

In einer erfindungsgemäßen Ausführungsform handelt es sich bei dem Filter um einen Keramikfilter oder um eine Polymermembran. Erfindungsgemäß bevorzugt handelt es sich bei dem Filter um einen Keramikfilter.

In einer erfindungsgemäßen Ausführungsform handelt es sich bei dem Filter um eine in der DE 101 54 549 A1 beschriebene Vorrichtung. Der Offenbarungsgehalt der DE 101 54 549 A1 ist vollständig in die vorliegende Beschreibung der Erfindung mit einbezogen. In einer erfindungsgemäßen Ausführungsform handelt es sich bei dem Filter um eine Vorrichtung zum Trennen von Stoffen, insbesondere Feststoffen, Flüssigkeitsphasen unterschiedlicher Dichte und/oder Gasen, aus einer Flüssigkeit, durch Rotation mit mehreren Filterelementen, welche die filtrierte Flüssigkeit passieren lassen und die um eine Drehachse drehbar in einem Gehäuse aufgenommen sind, das eine Eintrittsöffnung für die Flüssigkeit, mindestens eine Austrittsöffnung für die durch Rotation getrennten schweren Stoffe und mindestens eine Austrittsöffnung für die filtrierte Flüssigkeit aufweist, wobei im Bereich oder in der Nähe der Drehachse der Filterelemente ein Durchgang für Stoffe vorgesehen ist, die eine geringere Dichte als die Flüssigkeit aufweisen.

In einer erfindungsgemäßen Ausführungsform wird die anaerob biologisch gereinigte organische Suspension und/oder die mindestens eine wässrige Phase in einem zusätzlichen Schritt vor dem Zuführen in die Algenkultur filtriert. In einer erfindungsgemäßen Ausführungsform wird die anaerob biologisch gereinigte organische Suspension und/oder die mindestens eine wässrige Phase in einem zusätzlichen Schritt vor dem Zuführen in die Algenkultur mikrofiltriert oder ultrafiltriert. In einer erfindungsgemäßen Ausführungsform wird die anaerob biologisch gereinigte organische Suspension und/oder die mindestens eine wässrige Phase in einem zusätzlichen Schritt vor dem Zuführen in die Algenkultur mikrofiltriert. In einer erfindungsgemäßen Ausführungsform wird die anaerob biologisch gereinigte organische Suspension und/oder die mindestens eine wässrige Phase in einem zusätzlichen Schritt vor dem Zuführen in die Algenkultur ultrafiltriert.

Die Filtration dient insbesondere zum Abtrennen etwaiger organischer Feststoffe, insbesondere zum Abtrennen von Mikroorganismen. Dadurch wird eine Kontamination der Algenkultur mit anderen Mikroorganismen vermieden. Eine solche Kontamination könnte zum einen das Wachstum der Algen behindern und zum anderen eine Reinigung der Algenkultur nötig machen, um die Abwässer der AIgenkultur verklappen zu können. Auch könnte die Aufreinigung von Stoffen, die aus den Algen gewonnen werden sollen, erschwert werden.

Es kann in einer erfindungsgemäßen Ausführungsform vorgesehen sein, dass die beim Filtrieren vom Filtrat abgetrennten Konzentrate, die auch Gärreststoffe genannt werden, wieder einem anaeroben biologischen Reinigungsschritt zugeführt werden. Die Konzentrate können alternativ aber auch abgeleitet werden.

In einer erfindungsgemäßen Ausführungsform sind die Konzentrate dickflüssig.

Erfindungsgemäß bevorzugt ist die wässrige Phase fäkalkeimfrei. In einer Ausführungsform der Erfindung ist die wässrige Phase gänzlich keimfrei oder nahezu keimfrei. Erfindungsgemäß bevorzugt handelt es sich also bei der wässrigen Phase um ein fäkalkeimfreies, insbesondere keimfreies Filtrat. Erfindungsgemäß bevorzugt ist die wässrige Phase frei von Pilzen oder nahezu frei von Pilzen. Erfindungsgemäß bevorzugt ist die wässrige Phase frei von Viren oder nahezu frei von Viren. Damit kann eine keimarme Mikroalgenbiomasseproduktion gewährleistet werden, durch die die Mikroalgenbiomasse einer hochwertigen Verwertung zugeführt werden kann.

Es zeigte sich überraschender Weise, dass die wässrigen Phasen ohne Problem so aufgereinigt werden können, dass sie keine Bakterien enthalten oder zumindest Bakterien nur in so geringen Konzentrationen enthalten, dass die Bakterien weder zu einer Beeinträchtigung des Algenwachstums noch zu einer Gesundheitsgefährdung führen. Insbesondere zeigte sich überraschender Weise, dass Filtrate von anaerob biologisch gereinigten organischen Suspension nahezu frei von Bakterien sind oder sogar gänzlich frei von Bakterien sind.

Es zeigte sich darüber hinaus, dass die wässrigen Phasen, insbesondere Filtrate, toxische Substanzen aus dem Klärprozess in so geringen Konzentrationen enthalten, dass sie das Algenwachstum nicht behindern.

Es zeigte sich überraschenderweise, dass wässrige Phasen aus anaerob biologisch gereinigten organischen Suspensionen als Substitut und Teilsubstitut von Medien, beispielsweise Mineralsalzmedien zur Kultivierung von Algen, insbesondere Mikroalgen, eingesetzt werden können. Überraschenderweise führt die Verwendung von wässrigen Phasen aus anaerob biologisch gereinigten organischen Suspensionen als Medienbestandteil zu höheren Zuwachsraten der Algenbiomasse als die Verwendung herkömmlicher synthetischer Medien, die eine sehr gute Bereitstellung von Stickstoff aufweisen.

Durch das erfindungsgemäße Verfahren kann somit der Zusatz von Stickstoff zu Algenkulturen, der aufwendig, kostenintensiv und Energie verbrauchend durch das Haber-Bosch-Verfahren hergestellt wurde, reduziert werden oder sogar auf die Verwendung von solchem Stickstoff bei der Kultivierung von Algen ganz verzichtet werden.

Es zeigte sich überraschenderweise, dass wässrige Phasen aus anaerob biologisch gereinigten organischen Suspensionen durch ihre Zusammensetzung den Algen ausreichende, wenn nicht gar ideale Mengen an mineralischen Wertstoffen, insbesondere Stickstoff und Phosphor, zuführen. Ohne an die Theorie gebunden zu sein wird dabei vermutet, dass nicht nur die in den wässrigen Phasen vorhandenen Ammoniumkonzentrationen, sondern auch die verfügbaren Karbonatmengen das Wachstum der Algen stimulieren.

Erfindungsgemäß bevorzugt enthält die wässrige Phase der anaerob biologisch gereinigten organischen Suspension 0,5 bis 5 g/l Ammoniumionen, insbesondere 1 bis 3 g/l Ammoniumionen.

Erfindungsgemäß bevorzugt enthält die wässrige Phase der anaerob biologisch gereinigten organischen Suspension zwischen 1 und 500 mg/l Phosphat, insbesondere zwischen 10 und 200 mg/l Phosphat. In einer erfindungsgemäßen Ausführungsform enthält die wässrige Phase der anaerob biologisch gereinigten organischen Suspension mindestens 1 mg/l Phosphat. In einer erfindungsgemäßen Ausführungsform enthält die wässrige Phase der anaerob biologisch gereinigten organischen Suspension mindestens 10 mg/l Phosphat. In einer erfindungsgemäßen Ausführungsform enthält die wässrige Phase der anaerob biologisch gereinigten organischen Suspension höchstens 500 mg/l Phosphat. In einer erfindungsgemäßen Ausführungsform enthält die wässrige Phase der anaerob biologisch gereinigten organischen Suspension höchstens 200 mg/l Phosphat.

Es zeigte sich auch, dass durch die fakultativen heterotrophen Stoffwechsel der Algen auch der CSB (chemische Sauerstoffbedarf) in den wässrigen Phasen weiter reduziert werden kann.

Das erfindungsgemäße bioprozesstechnische Verfahren kann also beispielsweise insbesondere folgende Schritte enthalten:
a) anaerob biologisches Reinigen einer organischen Suspension,
b) Filtrieren der anaerob biologisch gereinigten organischen Suspension und
c) Zuführen des Filtrats aus der anaerob biologisch gereinigten organischen Suspension als Medienbestandteil in eine Algenkultur.

Im Zusammenhang mit der vorliegenden Erfindung wird unter einem Medienbestandteil einer Algenkultur ein bestimmter Anteil einer flüssigen Zusammensetzung verstanden, die sich zum Kultivieren von Algen, insbesondere von Mikroalgen, eignet. Der Anteil kann von 1 % bis 100 % des Algenkulturgesamtmediums betragen. Dem Fachmann sind verschiedene Algenkulturmedien aus dem Stand der Technik bekannt.

Erfindungsgemäß bevorzugt enthält das für die Algenkultur verwendete Gesamtmedium mindestens 50 Gew.-% an der wässrigen Phasen als Medienbestandteil.

Erfindungsgemäß bevorzugt enthält das für die Algenkultur verwendete Gesamtmedium mindestens 50 Gew.-% an den wässrigen Phasen. Erfindungsgemäß bevorzugt enthält das für die Algenkultur verwendete Gesamtmedium mindestens 60 Gew.-% an den wässrigen Phasen. In einer erfindungemäßen Ausführungsform enthält das für die Algenkultur verwendete Gesamtmedium mindestens 75 Gew.-% an den wässrigen Phasen. In einer erfindungsgemäßen Ausführungsform enthält das für die Algenkultur verwendete Gesamtmedium mindestens 80 Gew.-% an den wässrigen Phasen. In einer erfindungsgemäßen Ausführungsform enthält das für die Algenkultur verwendete Gesamtmedium mindestens 90 Gew.-% an den wässrigen Phasen. Das der Algenkultur zugeführte Gesamtmedium kann bis zu 99,9% die wässrige Phase enthalten. Das für die Algenkuttur verwendete Gesamtmedium kann auch aus der mindestens einen wässrigen Phase bestehen. Das für die Algenkultur verwendete Gesamtmedium kann auch nur aus den wässrigen Phasen bestehen.

Erfindungsgemäß kann es also vorgesehen sein, herkömmliches Algenkulturmedium durch die wässrige Phase ganz oder teilweise zu ersetzen. Erfindungsgemäß kann es also vorgesehen sein, herkömmliches Algenkulturmedium durch die wässrige Phase ganz zu ersetzen. Erfindungsgemäß kann es also vorgesehen sein, herkömmliches Algenkulturmedium durch die wässrige Phase teilweise, beispielsweise zu 50 %, zu 75 % oder zu 90 %, zu ersetzen.

Da die Zusammensetzung einer wässrigen Phase aus einer anaerob biologisch gereinigten organischen Suspension von der Zusammensetzung der Ausgangssuspension, beispielsweise von der Abwasserzusammensetzung abhängt, kann durch Zugabe von Spurenelementen die Wachstumsstimulierung bei Bedarf noch weiter verbessert werden.

Erfindungsgemäß kann auch vorgesehen sein, der mindestens einen wässrigen Phasen Spurenelemente zuzufügen. Erfindungsgemäß kann auch vorgesehen sein, der mindestens einen wässrigen Phasen Phosphor, beispielsweise als Phosphat zuzufügen.

Entsprechend jener Zusammensetzung des Mineralsalzmediums, welches maximale Produktwachstumsraten für eine spezifische Algenart garantiert, kann in einer erfindungsgemäßen Ausführungsform die Differenz von Inhaltsstoffen, insbesondere Phosphor, zwischen den erfindungsgemäß als Medium verwendeten wässrigen Phasen und optimalen Wachstumsmedien ausgeglichen werden.

Erfindungsgemäß kann auch vorgesehen sein, in der mindestens einen wässrigen Phase das Verhältnis zwischen Phosphor und Stickstoff auf etwa 3 zu 1, beispielsweise auf 2 zu 1 bis 4 zu 1, insbesondere auf 3 zu 1 einzustellen. Die Einstellung kann durch entsprechende Zugabe von Phosphor oder Stickstoff erfolgen.

Erfindungsgemäß bevorzugt ist die Algenkultur eine Mikroalgenkultur.

Erfindungsgemäß bevorzugt handelt es sich bei der Algenkultur um eine *Phaedodactylum tricomutum* Kultur, oder um eine *Haematococcus pluvialis* Kultur, oder um eine *Chlorella sorokiniana* Kultur, oder um eine *Chlorella vulgaris* Kultur, oder um eine *Platymonas subcordiformis* Kultur, oder um eine *Tetraseimis suecica* Kultur, oder um eine *Nannochloropsis oculata* Kultur, oder um eine *Isochrysis spec.* Kultur, oder um eine *Nannochloropsis limnetica* Kultur, oder um eine *Phormidium spec.* Kultur, oder um eine *Pseudoanabaena spec.* Kultur, oder um eine *Dunaliella spec.* Kultur, oder um eine *Monodus subterraneus* Kultur.

In einer erfindungemäßen Ausführungsform handelt es sich bei der Algenkultur um eine *Phaedodactylum tricomutum* Kultur. In einer erfindungemäßen Ausführungsform handelt es sich bei der Algenkultur um eine *Haematococcus pluvialis* Kultur. In einer erfindungemäßen Ausführungsform handelt es sich bei der Algenkultur um eine *Chlorella sorokiniana* Kultur. In einer erfindungemäßen Ausführungsform handelt es sich bei der Algenkultur um eine *Chlorella vulgaris* Kultur. In einer erfindungemäßen Ausführungsform handelt es sich bei der Algenkultur um eine *Platymonas subcordiformis* Kultur. In einer erfindungemäßen Ausführungsform handelt es sich bei der AIgenkultur um eine *Tetraseimis suecica* Kultur. In einer erfindungemäßen Ausführungsform handelt es sich bei der Algenkultur um eine *Nannochloropsis oculata* Kultur. In einer erfindungemäßen Ausführungsform handelt es sich bei der Algenkultur um eine *Isochrysis spec.* Kultur. In einer erfindungemäßen Ausführungsform handelt es sich bei der Algenkultur um eine *Nannochloropsis Iimnetica* Kultur. In einer erfindungemäßen Ausführungsform handelt es sich bei der Algenkultur um eine *Phormidium spec.* Kultur. In einer erfindungemäßen Ausführungsform handelt es sich bei der Algenkultur um eine *Pseudoanabaena spec.* Kultur. In einer erfindungemäßen Ausführungsform handelt es sich bei der Algenkultur um eine *Dunaliella spec.* Kultur. In einer erfindungemäßen Ausführungsform handelt es sich bei der Algenkultur um eine *Monodus subterraneus* Kultur.

In einer erfindungsgemäßen Ausführungsform wird die Algenkultur zur Herstellung von Wertstoffen, insbesondere von essentiellen Fettsäuren, Lipiden, Sterolen und Karotinoiden, Polysacchariden, Proteinen oder Aminosäuren und Phycobiliproteinen verwendet.

Die Algenkultur kann auch zur Gewinnung, also Herstellung von Biomasse als Rohstoff verwendet werden. Dabei wird die Biomasse durch die wachsende Algenmasse gebildet. Die Algenkultur kann auch zur Gewinnung von Protein-reicher, Nucleinsäure-armer Biomasse als Rohstoff verwendet werden. Die Algenkultur kann auch zur Gewinnung von Kohlenhydrat-reicher, Nucleinsäure-armer Biomasse als Rohstoff verwendet werden.

In einer erfindungsgemäßen Ausführungsform kann die Algenkultur, insbesondere die Algenkultur in einem Photobioreaktor, als Rohstoff für eine Biomassenraffinerie verwendet werden. In einer erfindungsgemäßen Ausführungsform kann vorgesehen sein, die Algenkultur in einem Photobioreaktor als Rohstoff für eine Biomassenraffinerie zu verwenden, bei der als erstes Produkt mindestens ein Wertstoff, insbesondere mindestens ein Wertstoff ausgewählt aus der Gruppe bestehend aus essentiellen Fettsäuren, Lipiden, Sterolen, Karotinoiden, Polysacchariden, Proteinen, Aminosäuren, Phycobiliproteinen oder Mischungen davon produziert wird und als zweites Produkt Biomasse produziert wird.

Erfindungsgemäß bevorzugt werden dabei 1 bis 10 % Wertstoff und 90 bis 99 % Biomasse produziert, insbesondere 4 bis 6 % Wertstoff und 94 bis 96 % Biomasse.

Somit können die erfindungsgemäß kultivierten Algenkulturen ein überraschend gutes Verhältnis an Wertstoff zu Biomasse produzieren.

In einer erfindungsgemäß bevorzugten Ausführungsform handelt es sich bei dem Wertstoff um mindestens eine Omega-3-Fettsäure, insbesondere um eine Omega-3-Fettsäure. Insbesondere kann es sich bei dem Wertstoff um Eicosapentaensäure (EPA) handeln. Es kann sich bei dem Wertstoff aber auch beispielsweise um Docosahexaensäure (DHA) handeln. In einer alternativen Ausführungsform kann es sich bei dem Wertstoff auch um ein Karotinoid, insbesondere um Lutein handeln.

Es kann also in einer erfindungsgemäßen Ausführungsform vorgesehen sein, dass als Edukt des erfindungsgemäßen Verfahrens die wässrige Phase aus mindestens einer anaerob biologisch gereinigten organischen Suspension, beispielsweise Abwasser oder Klärschlamm, verwendet wird und als Produkt ein Wertstoff erhalten wird, beispielsweise EPA oder Lutein.

Die Erfindung betrifft also auch ein erfindungsgemäßes Verfahren, insbesondere ein bioprozesstechnisches Verfahren, bei dem in einem Hauptverfahrensschritt mindestens eine wässrige Phase aus mindestens einer anaerob biologisch gereinigten organischen Suspension als Medienbestandteil einer Algenkultur zugeführt wird, zur Herstellung von Biomasse und/oder mindestens einem Wertstoff.

Die Erfindung betrifft also auch ein erfindungsgemäßes Verfahren in einer Ausgestaltungsform als Herstellverfahren.

Die Erfindung betrifft also auch ein erfindungsgemäßes Verfahren zur Herstellung von Biomasse aus einer Algenkultur und/oder zur Herstellung von Wertstoffen. Die Erfindung betrifft also auch ein erfindungsgemäßes Verfahren zur Herstellung von Biomasse aus einer Algenkultur. Die Erfindung betrifft also auch ein erfindungsgemäßes Verfahren zur Herstellung von Wertstoffen.

Die Erfindung betrifft auch die durch das erfindungsgemäße Verfahren hergestellten Biomassen und/oder Wertstoffe.

Die Algenkultur kann auch zur weiteren Aufreinigung von wässrigen Phasen von anaerob biologisch gereinigten organischen Suspensionen verwendet werden. Die Algenkultur kann insbesondere zur Reduktion von Stickstoff, Phosphor und/oder des chemischen Sauerstoffbedarfs (CSB) in wässrigen Phasen von anaerob biologisch gereinigten organischen Suspensionen verwendet werden.

Die Erfindung betrifft also auch ein erfindungsgemäßes Verfahren, insbesondere ein bioprozesstechnisches Verfahren, bei dem in einem Hauptverfahrensschritt mindestens eine wässrige Phase aus mindestens einer anaerob biologisch gereinigten organischen Suspension als Medienbestandteil einer Algenkultur zugeführt wird, zur Herstellung von gereinigten organischen Suspensionen mit einem reduzierten Gehalt an Stickstoff, Phosphor und/oder CSB.

Die Erfindung betrifft also auch ein erfindungsgemäßes Verfahren zur Reinigung von wässrigen Phasen anaerob biologisch gereinigter organischer Suspensionen.

Die wässrigen Phasen von anaerob biologisch gereinigten, organischen Suspension können zur Verbesserung der Wachstumsbedingungen von Algen, insbesondere von Mikroalgen in Photobioreaktoren, verwendet werden. Die wässrigen Phasen von anaerob biologisch gereinigten, organischen Suspension können auch zur Reduktion von Substrat-bedingten Betriebskosten bei der Kultivierung von Algen, insbesondere von Mikroalgen in Photobioreaktoren, verwendet werden.

Die Algenkultur kann auch zur Bindung von freiem CO₂ verwendet werden.

In einer Ausführungsform der Erfindung kann vorgesehen sein, der Algenkultur nicht nur die wässrige Phase aus mindestens einer anaerob biologisch gereinigten organischen Suspension als Medienbestandteil zuzuführen, sondern zusätzlich auch das bei der anaeroben biologischen Reinigung der organischen Suspension entstandene CO₂ der Algenkultur zuzuführen. Das erfindungsgemäße bioprozesstechnische Verfahren kann also beispielsweise insbesondere folgende Schritte enthalten:
a) anaerob biologisches Reinigen einer organischen Suspension,
b) Filtrieren der anaerob biologisch gereinigten organischen Suspension,
c) Zuführen des Filtrats aus der anaerob biologisch gereinigten organischen Suspension als Medienbestandteil in eine Algenkultur,
d) Zuführen des bei der anaeroben biologischen Reinigung entstandenen CO₂ in die Algenkultur.

Wahlweise kann das bei der anaeroben biologischen Reinigung entstandene Methan zur Energiegewinnung verbrannt werden und das bei der Verbrennung entstandene CO₂ ebenfalls der Algenkultur zugeführt werden.

Das erfindungsgemäße bioprozesstechnische Verfahren kann also beispielsweise insbesondere folgende Schritte enthalten:
a) anaerob biologisches Reinigen einer organischen Suspension,
b) Filtrieren der anaerob biologisch gereinigten organischen Suspension,
c) Zuführen des Filtrats aus der anaerob biologisch gereinigten organischen Suspension als Medienbestandteil in eine Algenkultur,
d) Zuführen des bei der anaeroben biologischen Reinigung entstandenen CO₂ in eine Algenkultur,
e) Verbrennen des bei der anaeroben biologischen Reinigung entstandenen Methan zur Energiegewinnung,
f) Zuführen des bei der Methanverbrennung entstandenen CO₂ in die Algenkultur.

Das erfindungsgemäße bioprozesstechnische Verfahren kann also beispielsweise insbesondere folgende Schritte enthalten:
a) anaerob biologisches Reinigen einer organischen Suspension,
b) Filtrieren der anaerob biologisch gereinigten organischen Suspension,
c) Zuführen des Filtrats aus der anaerob biologisch gereinigten organischen Suspension als Medienbestandteil in eine Algenkultur,
d) Verbrennen des bei der anaeroben biologischen Reinigung entstandenen Biogas zur Energiegewinnung,
e) Zuführen des bei der Methanverbrennung entstandenen CO₂ in die Algenkultur..

Die vorliegende Erfindung stellt also ein Verfahren bereit, bei dem eine Kreislaufführung eines CO₂-sorbierenden Prozesses möglich ist. Im vorgeschalteten Verfahrensschritt der anorganischen biologischen Reinigung entstehen als Endprodukte eine wässrige Phase und bei der Vergärung und bei der Verbrennung des entstehenden Methan und der entstehenden Biomasse CO₂. In dem nachgeschalteten Verfahrensschritt, nämlich der Kultivierung der Algen, können diese Produkte, also die wässrige Phase und das CO₂ als Edukte eingesetzt werden, insbesondere auch vollständig eingesetzt werden. Aus diesen Edukten entsteht durch die Kultivierung der Algen wahlweise ein Wertstoff und dazu insbesondere Biomasse. Diese Biomasse kann wiederum als Edukt verwendet werden, wobei nach einer energetischen Verwertung und nachfolgender Aufreinigung eine wässrige Phase und CO₂ entstehen. Somit kann das erfindungsgemäße Verfahren in einem Kreislauf eingesetzt werden, bei dem gebildete Energie und Wertstoffe abgezweigt werden können.

Darüber hinaus werden bei dem erfindungsgemäßen Verfahren Betriebskosten bei der Kultivierung von Algen, insbesondere in Photobioreaktoren, eingespart, da die Edukte CO₂ und Nährstoffe in Form der wässrigen Phase durch die anaerobe biologische Reinigung der organischen Suspension entstehen. So kann zum Beispiel in einer Fabrik die dort entstehenden Abwässer in aufgereinigter Form als Kulturmedien für Algenkulturen verwendet werden, während das durch Verbrennungsprozesse in der Fabrik entstehende CO₂ ebenfalls diesen Algenkulturen zugeleitete werden kann.

Somit ist eine kostengünstige und zugleich umweltschonende Verwendung der wässrigen Phasen von anaerob biologisch gereinigten organischen Suspensionen, insbesondere von Abwässern oder Klärschlämmen, möglich.

Erfindungsgemäß bevorzugt wird die Algenkultur in einem Photobioreaktor kultiviert.

Der Fachmann kennt Photobioreaktoren, die sich zur Kultivierung von Algen, insbesondere Mikroalgen, eignen.

In einer erfindungsgemäßen Ausführungsform ist der Photobioreaktor ein in der DE 199 16 597 A1 beschriebener Photobioreaktor. Der Offenbarungsgehalt der DE 199 16 597 A1 ist vollständig in die vorliegende Beschreibung der Erfindung mit einbezogen.

Erfindungsgemäß bevorzugt ist ein Photobioreaktor, der einen Reaktorraum aus lichtdurchlässigem Material aufweist. Erfindungsgemäß bevorzugt ist ein Photobioreaktor, dessen Reaktorraum eine Oberflächenvergrößerung größer als die geradflächige umhüllende Fläche eines Volumens aufweist. Erfindungsgemäß bevorzugt ist ein Photobioreaktor, der Einrichtungen für eine turbulente Strömungsführung aufweist. Erfindungsgemäß bevorzugt ist ein Photobioreaktor, der Elemente aufweist, die Licht von außen in den Reaktorraum leiten.

Die vorliegende Erfindung betrifft auch die Verwendung von wässrigen Phasen von anaerob biologisch gereinigten organischen Suspensionen als Medienbestandteil einer Algenkultur, insbesondere einer Mikroalgenkultur.

Die vorliegende Erfindung betrifft auch die Verwendung von wässrigen Phasen von anaerob biologisch gereinigten organischen Suspensionen zur Verbesserung der Wachstumsbedingungen von Algen, insbesondere Mikroalgen, in Photobioreaktoren.

Die vorliegende Erfindung betrifft auch die Verwendung von Algen, insbesondere von Mikroalgen, zur Reinigung von wässrigen Phasen von anaerob biologisch gereinigten organischen Suspensionen, insbesondere von anaerob biologisch gereinigtem Abwasserfiltrat.

Die vorliegende Erfindung betrifft auch die durch die erfindungsgemäße Kultivierung der Algen gewonnenen Produkte.

Die vorliegende Erfindung betrifft auch eine bioprozesstechnische Vorrichtung, enthaltend einen Bioreaktor, beispielsweise einen Faulturm, und einen Photobioreaktor.

Bei dem Bioreaktor handelt es sich insbesondere um einen Reaktor, der zur anearoben biologischen Reinigung von Suspensionen geeignet ist, also um einen Anaerobreaktor.

Erfindungsgemäß bevorzugt enthält die Vorrichtung eine Filtrationsvorrichtung.

Erfindungsgemäß ist die Filtrationsvorrichtung eine Mikrofiltrationsvorrichtung.

Erfindungsgemäß bevorzugt weist die Filtrationsvorrichtung oder die Mikrofiltrationsvorrichtung Mittel zum Entfernen von Feststoffen auf.

Erfindungsgemäß bevorzugt weist der Bioreaktor eine Zuleitung zu der Filtrationsvorrichtung auf und die Filtrationsvorrichtung weist eine Zuleitung zu dem Photobioreaktor auf.

Erfindungsgemäß bevorzugt weist der Bioreaktor eine Flüssigkeitszuleitung zu der Filtrationsvorrichtung auf. Erfindungsgemäß bevorzugt weist die Filtrationsvorrichtung eine Flüssigkeitszuleitung zu dem Photobioreaktor auf. Erfindungsgemäß bevorzugt weist die Filtrationsvorrichtung eine Zuleitung zum Bioreaktor zur Überführung der Konzentrate, auch Gärreststoffe genannt, auf, die in der Filtrationsvorrichtung von dem Filtrat abgeschieden werden. Erfindungsgemäß bevorzugt kann auch alternativ oder insbesondere zusätzlich vorgesehen sein, dass die Filtrationsvorrichtung eine Ableitung für die Konzentrate, also Gärreststoffe aufweist.

Eine bevorzugte Ausführungsform ist eine bioprozesstechnische Vorrichtung, enthaltend einen Bioreaktor, eine Filtrationsvorrichtung und einen Photobioreaktor, wobei der Bioreaktor eine Flüssigkeitszuleitung zu der Filtrationsvorrichtung aufweist und die Filtrationsvorrichtung eine Flüssigkeitszuleitung zu dem Photobioreaktor aufweist.

Eine bevorzugte Ausführungsform ist eine bioprozesstechnische Vorrichtung, enthaltend einen Bioreaktor, eine Filtrationsvorrichtung und einen Photobioreaktor, wobei der Bioreaktor eine Flüssigkeitszuleitung zu der Filtrationsvorrichtung aufweist und die Filtrationsvorrichtung eine Flüssigkeitszuleitung zu dem Photobioreaktor aufweist und wobei die Filtrationsvorrichtung Mittel zum Entfernen von Feststoffen aufweist und/oder wobei die Filtrationsvorrichtung eine Feststoffzuleitung zum Bioreaktor zur Überführung der Konzentrate aufweist.

Erfindungsgemäß bevorzugt weist der Bioreaktor eine Gasableitung zum Ableiten von bei der Vergärung entstehenden Gasen, beispielsweise Biogas, Methan und/oder CO₂, auf.

Erfindungsgemäß bevorzugt weist der Photobioreaktor eine Gaszuleitung zum Zuleiten von CO₂, auf.

In einer erfindungemäßen Ausführungsform weißt die die Vorrichtung einen Brennraum zum Verbrennen des im Bioreaktor entstandenen Biogases oder Methans auf. Erfindungsgemäß bevorzugt ist der Brennraum mit dem Bioreaktor über die Gasableitung und mit dem Photobioreaktor über die Gaszuleitung verbunden. Erfindungsgemäß bevorzugt dient das Verbrennen der Gase im Brennraum der Energiegewinnung.

Erfindungsgemäß bevorzugt weist der Bioreaktor Vorrichtungen zum Durchmischen des Reaktorinhalts auf.

Die vorliegende Erfindung betrifft auch die erfindungsgemäße bioprozesstechnische Vorrichtung in einer speziellen Ausbildung zur Durchführung des erfindungsgemäßen Verfahrens.

Die vorliegende Erfindung betrifft auch die Verwendung einer erfindungsgemäßen bioprozesstechnischen Vorrichtung zur Durchführung des erfindungsgemäßen Verfahrens.

Vorliegende Beschreibungen zu erfindungsgemäßen alternativen und/oder bevorzugten Ausführungsformen des erfindungsgemäßen Verfahrens beziehen sich auch auf Ausführungsformen der erfindungsgemäßen Verwendungen, erfindungsgemäßen Produkte und erfindungsgemäßen Vorrichtungen.

Vorliegende Beschreibungen zu erfindungsgemäßen alternativen und/oder bevorzugten Ausführungsformen der erfindungsgemäßen Verwendungen beziehen sich auch auf Ausführungsformen der erfindungsgemäßen Verfahren, erfindungsgemäßen Produkte und erfindungsgemäßen Vorrichtungen.

Vorliegende Beschreibungen zu erfindungsgemäßen alternativen und/oder bevorzugten Ausführungsformen der erfindungsgemäßen Vorrichtungen beziehen sich auch auf Ausführungsformen der erfindungsgemäßen Verwendungen, erfindungsgemäßen Produkte und erfindungsgemäßen Verfahren.

Weiter Ausführungsformen der vorliegenden Erfindung finden sich in den Unteransprüchen.

Die Erfindung wird an folgendem Beispiel und den Figuren näher erläutert:
Figur 1 zeigt eine Ausführungsform einer erfindungsgemäßen Vorrichtung, enthaltend einen Bioreaktor, eine Filtrationsvorrichtung und einen Photobioreaktor.
Figur 2 zeigt Vergleiche der Biomasseproduktivität von *Phaeodactylum tricomutum* mit verschiedenen N-Quellen in Abhängigkeit von der relativen Lichtverfügbarkeit (Figur 2A) und der Trockensubstanz (TS) -Konzentration im Reaktor (Figur 2B).
Figur 3 zeigt das Wachstum von *Phaeodactylum tricomutum* in einem Flachplatten-Airlift-Photobioreaktor (FPA)-Reaktor bei einer Lichtintensität von 400 µE m⁻² s⁻¹.

### Beispiel:

### Vorrichtung zur Durchführung des erfindungsgemäßen Verfahrens

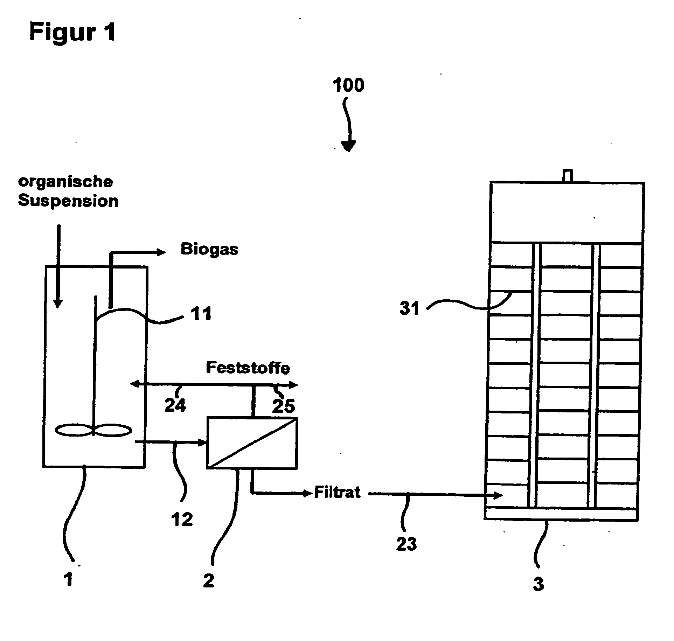

Eine beispielhafte Ausführungsform einer erfindungsgemäßen Vorrichtung (100) ist in Figur 1 gezeigt. Die Vorrichtung setzt sich aus einem Bioreaktor (1), einer Filtrationsvorrichtung (2) und einem Photobioreaktor (3) zusammen. Der Bioreaktor (1) und die Filtrationsvorrichtung (2) sind über eine Leitung (12) miteinander verbunden. Auch die Filtrationsvorrichtung (2) und der Photobioreaktor (3) sind über eine Leitung (23) miteinander verbunden.

Der Bioreaktor (1) kann beispielsweise als Faulturm ausgestaltet sein und Vorrichtungen zum Durchmischen des Reaktorinhalts, zum Beispiel eine Rührvorrichtung (11) aufweisen. Im Bioreaktor (1) werden organische Suspensionen, zum Beispiel Abwasser, anaerob biologisch gereinigt. Das dabei entstehende Biogas kann abgeleitet werden und zur Energiegewinnung genutzt werden. CO₂, das bei der Energiegewinnung aus dem Biogas entsteht, kann dem Photobioreaktor (3) zugeführt werden, wo das CO₂ durch Photosynthese der im Photobioreaktor (3) enthaltenen Algen gebunden wird. Die anaerob biologisch gereinigte organische Suspension, zum Beispiel Abwasser, wird über die Flüssigkeitszuleitung (12) zu der Filtrationsvorrichtung (2), beispielsweise einem Rotationsscheibenfilter, einem Polymerfilter oder einem keramischen Tiefenfilter, geführt. Dort findet eine weitere Aufreinigung statt, bei der Feststoffe entfernt werden. Die Feststoffe, auch Konzentrat oder Gärreststoffe genannt, können entweder über die Zuleitung (24) wieder dem Bioreaktor (1) zugeführt werden, oder sie werden über Ableitung (25) aus dem System entfernt. Das Filtrat wird über die Flüssigkeitszuleitung (23) zu dem Photobioreaktor (3) geführt. In dem Photobioreaktor (3) sind Algen, beispielsweise Mikroalgen, kultiviert. Bei dem Photobioreaktor (3) kann es sich beispielsweise um einen Flachplatten-Airlift-Reaktor (FPA) handeln, der durch seine spezielle Konstruktion mit statischen Mischern (31) eine höhere Produktivität bei hohen Zellkonzentrationen erlaubt.

Das zugeführte Filtrat wird von den im Photobioreaktor (3) kultivierten Algen als Medium verwendet. Dies führt zu einem besonders guten Algenwachstum und gleichzeitig zur weiteren Aufreinigung des Filtrats, da diesem Phosphat und Stickstoff entzogen wird.

Die Algen können zur Biomassengewinnung oder zur Gewinnung spezifischer Rohstoffe verwendet werden.

### Analyse einer Filtratprobe aus einem Anaerobprozess

Eine Filtratprobe aus einer zweistufigen Hochlastfaulung für Klärschlamm (TBB 6.12.08/29.4.08) wurde auf ihren Gehalt an Phosphat, Ammoniumstickstoff und CSB getestet. Die Analyse ergab folgende Werte:

| | |
|---|---|
| P04-P: | 26,25 mg/l |
| Ammoniumstickstoff: | 855 mg/l |
| CSB: | 200 mg/l |
| pH: | 7,8 |
| Wasserhärte | 14,2 °dH |
| Ca: | 71,8 mg/l |
| Mg: | 17,8 mg/l |

Bei einem durchschnittlichen NH₄-Bedarf von 30 mg/OD (optische Dichte) für das Wachstum der Mikroalge *Phaedodactylum tricomutum* kann unter Verwendung von Abwasserfiltrat als Medienbestandteil bei 20% Medienaustausch ein OD-Anstieg um 7,8 Einheiten pro Tag in der Algenkultur erreicht werden.

### Kultivierung von Mikroalgen mit verschiedenen bekannter Stickstoffquellen im Vergleich mit einer Filtratprobe aus einem Anaerobprozess

Mikroalgen der Gattung *Phaedodactylum tricomutum* wurden in einem FPA-Photobioreaktor kultiviert. Zu verschiedenen Zeitpunkten wurde die den Mikroalgen zur Verfügung stehende Stickstoffquelle gewechselt. In den ersten neun Tagen diente Ammoniumchlorid als Stickstoffquelle. In den Tagen 10 bis 43 diente Ammoniumcarbonat als Stickstoffquelle. In den Tagen 44 bis 60 diente die oben genannte Filtratprobe aus einem Anaerobprozess als Stickstoffquelle. In den Tagen 61 bis 74 diente Ammoniumcarbonat als Stickstoffquelle. Auch wurde Harnstoff als Stickstoffquelle eingesetzt.

Die Produktionsraten der Algenkultur wurden über verschiedene Zeiträume gemessen.

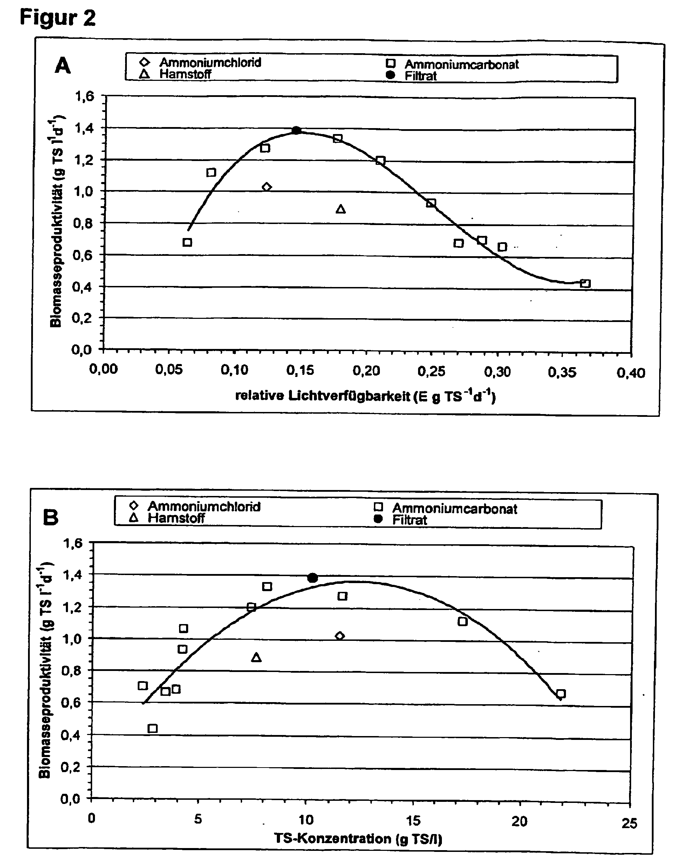

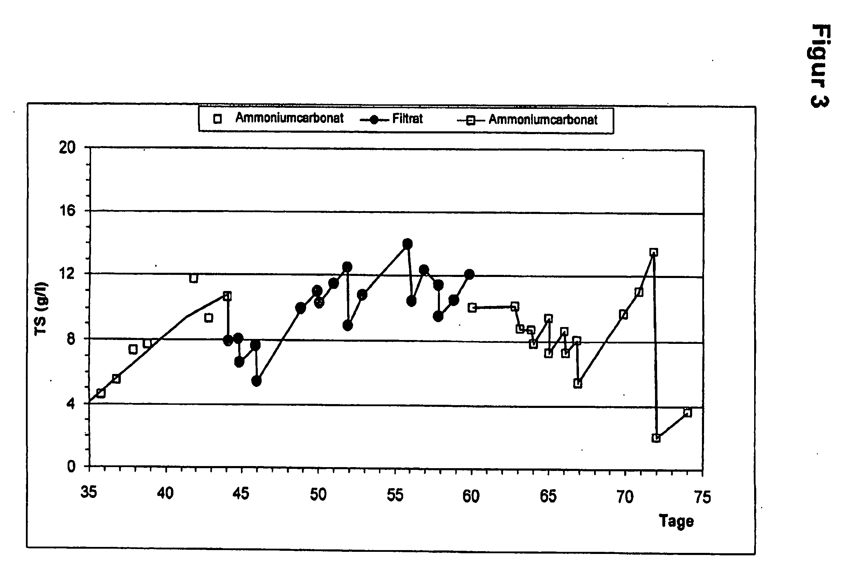

Die Versuchsergebnisse wurden in den Figuren 2 und 3 zusammengefasst. In den Figuren 2A und 2B ist die polynomische Trendlinie der Messpunkte für Ammoniumcarbonat eingezeichnet. Die Messpunkte für das Abwasser liegen oberhalb dieser Trendlinie.

Es zeigte sich, dass mit der Verwendung der Filtratprobe eines Anaerobprozesses als Stickstoffquelle die besten Ergebnisse erzielt wurden. Es konnte durch die Verwendung der Filtratprobe eine hohe Biomassenproduktivität bei gleichzeitig hoher Trockensubstanzkonzentration erreicht werden, obwohl die relative Lichtverfügbarkeit eher gering war.

## Patentansprüche

1. Bioprozesstechnisches Verfahren, enthaltend den Schritt: Zuführen mindestens einer wässrigen Phase mindestens einer anaerob biologisch gereinigten organischen Suspension als Medienbestandteil in eine Algenkultur, wobei die anaerob biologisch gereinigte organische Suspension und/oder die wässrige Phase in einem zusätzlichen Schritt vor dem Zuführen in die Algenkultur mit einem Rotationsscheibenfilter, einem Polymerfilter oder einem keramischen Tiefenfilter zum Abtrennen etwaiger organischer Feststoffe mindestens einmal gefiltert werden und wobei die Algenkultur in einem Photobioreaktor kultiviert wird.

2. Verfahren nach Anspruch 1, wobei die wässrige Phase in einem in einem zusätzlichen Schritt vor dem Zuführen in eine Algenkultur aus der anaerob biologisch gereinigten organischen Suspension abgetrennt wird.

3. Verfahren nach einem der vorhergehenden Ansprüche, wobei die wässrige Phase aus einem Methangärungsprozess stammt.

4. Verfahren nach einem der vorhergehenden Ansprüche, wobei die anaerob biologisch gereinigte organische Suspension und/oder die wässrige Phase in dem zusätzlichen Schritt vor dem Zuführen in die Algenkultur mit einem dynamischen Rotationsscheibenfilter, einem dynamischen Polymerfilter oder einem keramischen Tiefenfilter mindestens einmal gefiltert werden.

5. Verfahren nach einem der vorhergehenden Ansprüche, wobei es sich bei der ungereinigten organischen Suspension um Klärschlamm, Biomüll, Abwässer aus der Lebensmittelindustrie, Abwässer aus der kommunalen Abwasserentsorgung und/oder Abwässer aus der Vergärung nachwachsender Rohstoffe handelt.

6. Verfahren nach einem der vorhergehenden Ansprüche, wobei das für die Algenkultur verwendete Gesamtmedium mindestens 50 Gew.-% an der wässrigen Phase als Medienbestandteil enthält.

7. Verfahren nach einem der vorhergehenden Ansprüche, wobei die Algenkultur eine Mikroalgenkultur ist.

8. Verfahren nach einem der vorhergehenden Ansprüche, wobei es sich bei der Algenkultur um eine *Phaedodactylum tricomutum* Kultur, oder um eine *Haematococcus pluvialis* Kultur, oder um eine *Chlorella sorokiniana* Kultur, oder um eine *Chlorella vulgaris* Kultur, oder um eine *Platymonas subcordiformis* Kultur, oder um eine *Tetraselmis suecica* Kultur, oder um eine *Nannochloropsis oculata* Kultur, oder um eine *Nannochlompsis limnetica* Kultur, oder um eine *Phormidium spec.* Kultur, oder um eine *Pseudoanabaena spec.* Kultur, oder um eine *Dunaliella spec.* Kultur, oder um eine *Monodus subterraneus* Kultur oder um eine *Isochrysis spec*. Kultur handelt.

9. Verfahren nach einem der vorhergehenden Ansprüche zur Herstellung von Biomasse aus einer Algenkultur und/oder zur Herstellung von Wertstoffen und/oder zur Reinigung von wässrigen Phasen anaerob biologisch gereinigter organischer Suspensionen.

10. Verwendung einer wässrigen Phase einer anaerob biologisch gereinigten organischen Suspension als Medienbestandteil einer in einem Photobioreaktor kultivierten Algenkultur, wobei die anaerob biologisch gereinigte organische Suspension und/oder die wässrige Phase in einem zusätzlichen Schritt vor dem Zuführen in die Algenkultur mit einem Rotationsscheibenfilter, einem Polymerfilter oder einem keramischen Tiefenfilter zum Abtrennen etwaiger organischer Feststoffe mindestens einmal gefiltert werden.

11. Verwendung einer wässrigen Phase einer anaerob biologisch gereinigten organischen Suspension zur Verbesserung der Wachstumsbedingungen von Algen in Photobioreaktoren, wobei die anaerob biologisch gereinigte organische Suspension und/oder die wässrige Phase in einem zusätzlichen Schritt vor dem Zuführen in die Algenkultur mit einem Rotationsscheibenfilter, einem Polymerfilter oder einem keramischen Tiefenfilter zum Abtrennen etwaiger organischer Feststoffe mindestens einmal gefiltert werden.

12. Verwendung von in einem Photobioreaktor kultivierten Algen zur Reinigung einer wässrigen Phase einer anaerob biologisch gereinigten organischen Suspension, insbesondere von anaerob biologisch gereinigtem Abwasserfiltrat, wobei die anaerob biologisch gereinigte organische Suspension und/oder die wässrige Phase in einem zusätzlichen Schritt vor dem Zuführen in die Algenkultur mit einem Rotationsscheibenfilter, einem Polymerfilter oder einem keramischen Tiefenfilter zum Abtrennen etwaiger organischer Feststoffe mindestens einmal gefiltert werden.

13. Bioprozesstechnische Vorrichtung (100), enthaltend einen Bioreaktor (1), eine Filtrationsvorrichtung (2) und einen Photobioreaktor (3), wobei der Bioreaktor (1) eine Flüssigkeitszuleitung (12) zu der Filtrationsvorrichtung (2) aufweist und die Filtrationsvorrichtung (2) eine Flüssigkeitszuleitung (23) zu dem Photobioreaktor (3) aufweist, wobei die Filtrationsvorrichtung (2) ein Rotationsscheibenfilter, ein Polymerfilter oder ein keramischer Tiefenfilter zum Abtrennen etwaiger organischer Feststoffe ist.

14. Vorrichtung (100) nach Anspruch 13, wobei die Filtrationsvorrichtung (2) Mittel (25) zum Entfernen von Feststoffen aufweist.

15. Verwendung einer Vorrichtung (100) nach einem der Ansprüche 13 bis 14 zur Durchführung eines Verfahrens nach einem der Ansprüche 1 bis 9.

## Claims

1. Bioprocess engineering method comprising the step: supply of at least one aqueous phase of at least one anaerobic biologically purified organic suspension as media component to an algal culture, wherein the anaerobic biologically purified organic suspension and/or the aqueous phase are filtered at least once by means of a rotating disk filter, a polymer filter or a ceramic depth filter in an additional step before being supplied to the algal culture, and wherein the algal culture is cultivated in a photobioreactor.

2. Method according to claim 1, wherein the aqueous phase is separated from the anaerobic biologically purified organic suspension in an additional step before being supplied to an algal culture.

3. Method according to any one of the preceding claims, wherein the aqueous phase is derived from a methane fermentation process.

4. Method according to any one of the preceding claims, wherein the anaerobic biologically purified organic suspension and/or the aqueous phase are filtered at least once by means of a dynamic rotating disk filter, a dynamic polymer filter or a ceramic depth filter in the additional step before being supplied to the algal culture.

5. Method according to any one of the preceding claims, wherein the unpurified organic suspension is sewage sludge, organic waste, wastewater from the food industry, effluent from municipal wastewater and/or wastewater from the fermentation of renewable raw materials.

6. Method according to any one of the preceding claims, wherein the total medium used for the algal culture contains at least 50% by weight of the aqueous phase as media component.

7. Method according to any one of the preceding claims, wherein the algal culture is a microalgal culture.

8. Method according to any one of the preceding claims, wherein the algal culture is a *Phaeodactylum tricornutum* culture, or a *Haematococcus pluvialis* culture, or a *Chlorella sorokiniana* culture, or a *Chlorella vulgaris* culture, or a *Platymonas subcordiformis* culture, or a *Tetraselmis suecica* culture, or a *Nannochloropsis oculata* culture, or a *Nannochloropsis limnetica* culture, or a *Phormidium* sp. culture, or a *Pseudoanabaena* sp. culture, or a *Dunaliella* sp. culture, or a *Monodus subterraneus* culture, or an *Isochrysis* sp. culture.

9. Method according to any one of the preceding claims for the manufacture of biomass derived from an algal culture and/or for the manufacture of valuable substances and/or for the purification of aqueous phases of anaerobic biologically purified organic suspensions.

10. Use of an aqueous phase of an anaerobic biologically purified organic suspension as media component of an algal culture cultivated in a photobioreactor, wherein the anaerobic biologically purified organic suspension and/or the aqueous phase are filtered at least once by means of a rotating disk filter, a polymer filter or a ceramic depth filter in an additional step before being supplied to the algal culture for separating possible organic solid matter.

11. Use of an aqueous phase of an anaerobic biologically purified organic suspension to improve the growth conditions of algae in photobioreactors, wherein the anaerobic biologically purified organic suspension and/or the aqueous phase are filtered at least once by means of a rotating disk filter, a polymer filter or a ceramic depth filter in an additional step before being supplied to the algal culture for separating possible organic solid matter.

12. Use of algae cultivated in a photobioreactor for the purification of an aqueous phase of an anaerobic biologically purified organic suspension, in particular of anaerobic biologically purified sewage filtrate, wherein the anaerobic biologically purified organic suspension and/or the aqueous phase are filtered at least once by means of a rotating disk filter, a polymer filter or a ceramic depth filter in an additional step before being supplied to the algal culture for separating possible organic solid matter.

13. A bioprocess engineering device (100), comprising a bioreactor (1), a filtration device (2) and a photobioreactor (3), wherein the bioreactor (1) has a fluid supply line (12) to the filtration device (2) and the filtration device (2) has a fluid supply line (23) to the photobioreactor (3), wherein the filtration device (2) is a rotating disk filter, a polymer filter or a ceramic depth filter for separating possible organic solid matter.

14. Device (100) according to claim 13, wherein the filtration device (2) comprises means (25) for removing solid matter.

15. Use of a device (100) according to any one of claims 13 to 14 for execution of a method according to any one of claims 1 to 9.

## Revendications

1. Procédé technique de traitement biologique, comportant l'étape : Introduction dans une culture d'algues en tant que constituant du milieu d'au moins une phase aqueuse issue d'au moins une suspension organique purifiée biologiquement de manière anaérobie, la suspension organique purifiée biologiquement de manière anaérobie et/ou la phase aqueuse étant filtrées au moins une fois lors d'une étape supplémentaire avant l'introduction dans la culture d'algues avec un filtre rotatif à disques, un filtre polymère ou un filtre céramique à lit profond pour séparer d'éventuelles matières solides organiques et la culture d'algues étant cultivée dans un photobioréacteur.

2. Procédé selon la revendication 1, dans lequel la phase aqueuse est séparée de la suspension organique purifiée biologiquement de manière anaérobie dans une étape supplémentaire, avant l'introduction dans une culture d'algues.

3. Procédé selon l'une des revendications précédentes, dans lequel la phase aqueuse est issue d'un processus de fermentation méthanique.

4. Procédé selon l'une des revendications précédentes, dans lequel la suspension organique purifiée biologiquement de manière anaérobie et/ou la phase aqueuse sont filtrées au moins une fois à l'aide d'un filtre rotatif à disques dynamique, d'un filtre polymère dynamique ou d'un filtre céramique à lit profond pendant l'étape supplémentaire avant l'introduction dans la culture d'algues.

5. Procédé selon l'une des revendications précédentes, dans lequel la suspension organique non purifiée correspond à des boues d'épuration, des biodéchets, des eaux usées de l'industrie agroalimentaire, des eaux usées des infrastructures sanitaires communales et/ou des eaux usées issues de la fermentation de matières premières renouvelables.

6. Procédé selon l'une des revendications précédentes, dans lequel le milieu global utilisé pour la culture d'algues contient au moins 50 % en poids de la phase aqueuse en tant que constituant du milieu.

7. Procédé selon l'une des revendications précédentes, dans lequel la culture d'algues est une culture de micro-algues.

8. Procédé selon l'une des revendications précédentes, dans lequel la culture d'algues est une culture de *Phaedodactylum tricomutum*, ou une culture d'*Haematococcus pluvialis*, ou une culture de *Chlorella sorokiniana,* ou une culture de *Chlorella varlgaris*, ou une culture de *Platymonas subcordiformis*, ou une culture de *Tetraselmis sarecica*, ou une culture de *Nannochloropsis ocarlata*, ou une culture de *Nannochloropsis limnetica*, ou une culture de *Phormidium spec.,* ou une culture de *Pseudoanabaena spec.,* ou une culture de *Dunaliella spec.,* ou une culture de *Monodus subterraneus* ou une culture de *Isochrysis spec.*

9. Procédé selon l'une des revendications précédentes, pour la fabrication de biomasse à partir d'une culture d'algues et/ou pour la fabrication de matières valorisables et/ou pour la purification de phases aqueuses de suspensions organiques purifiées biologiquement de manière anaérobie.

10. Utilisation d'une phase aqueuse d'une suspension organique purifiée biologiquement de manière anaérobie en tant que constituant du milieu d'une culture d'algues cultivée dans un photobioréacteur, la suspension organique purifiée biologiquement de manière anaérobie et/ou la phase aqueuse étant filtrées au moins une fois lors d'une étape supplémentaire avant l'introduction dans la culture d'algues avec un filtre rotatif à disques, un filtre polymère ou un filtre céramique à lit profond pour séparer d'éventuelles matières solides organiques.

11. Utilisation d'une phase aqueuse d'une suspension organique purifiée biologiquement de manière anaérobie pour l'amélioration des conditions de croissance d'algues dans des photobioréacteurs, la suspension organique purifiée biologiquement de manière anaérobie et/ou la phase aqueuse étant filtrées au moins une fois lors d'une étape supplémentaire avant l'introduction dans la culture d'algues avec un filtre rotatif à disques, un filtre polymère ou un filtre céramique à lit profond pour séparer d'éventuelles matières solides organiques.

12. Utilisation d'algues cultivées dans un photobioréacteur pour la purification d'une phase aqueuse d'une suspension organique purifiée biologiquement de manière anaérobie, en particulier d'un filtrat d'eau usée purifié biologiquement de manière anaérobie, la suspension organique purifiée biologiquement de manière anaérobie et/ou la phase aqueuse étant filtrées au moins une fois lors d'une étape supplémentaire avant l'introduction dans la culture d'algues avec un filtre rotatif à disques, un filtre polymère ou un filtre céramique à lit profond pour séparer d'éventuelles matières solides organiques.

13. Dispositif technique de traitement biologique (100), comportant un bioréacteur (1), un dispositif de filtration (2) et un photobioréacteur (3), le bioréacteur (1) comportant une conduite d'alimentation de liquide (12) qui chemine vers le dispositif de filtration (2) et ledit dispositif de filtration (2) comportant une conduite d'alimentation de liquide (23) qui chemine vers le photobioréacteur (3), le dispositif de filtration (2) étant un filtre rotatif à disques, un filtre polymère ou un filtre céramique à lit profond pour séparer d'éventuelles matières solides organiques.

14. Dispositif (100) selon la revendication 13, dans lequel le dispositif de filtration (2) comporte des moyens (25) pour l'élimination de matières solides.

15. Utilisation d'un dispositif (100) selon l'une des revendications 13 à 14 pour l'exécution d'un procédé selon l'une des revendications 1 à 9.
